(19) Europäisches Patentamt
European Patent Office
Office européen des brevets

(11) Veröffentlichungsnummer: **0 189 094**
**A2**

# (12) EUROPÄISCHE PATENTANMELDUNG

(21) Anmeldenummer: **86100421.6**

(22) Anmeldetag: **14.01.86**

(51) Int. Cl.[4]: **C07D 213/20** , A61K 31/44 , //C07D303/22,C07D303/34,C07-D303/36

(30) Priorität: **16.01.85 CH 187/85**

(43) Veröffentlichungstag der Anmeldung: **30.07.86 Patentblatt 86/31**

(84) Benannte Vertragsstaaten: **AT BE CH DE FR GB IT LI LU NL SE**

(71) Anmelder: **F. HOFFMANN-LA ROCHE & CO. Aktiengesellschaft**

**CH-4002 Basel(CH)**

(72) Erfinder: **Cassal, Jean-Marie, Dr.**
**4 rue du Markstein**
**F-68100 Mulhouse(FR)**
Erfinder: **Edenhofer, Albrecht, Dr.**
**Rudolf Wackernagelstrasse 35**
**CH-4125 Riehen(CH)**
Erfinder: **Ramuz, Henri, Prof. Dr.**
**Rheinparkstrasse 3**
**CH-4127 Birsfelden(CH)**

(74) Vertreter: **Lederer, Franz, Dr. et al**
**Patentanwälte Dr. Lederer Franz Meyer-Roxlau Reiner F. Lucile-Grahn-Strasse 22**
**D-8000 München 80(DE)**

## (54) Polycyclische Salze.

(57) Polycyclische Salze der Formel

$A^{-}XN^{+}-CH_2-(Y)_n-(CH_2)_p-(Z)_q-L-(T)_r-M$ I

worin

$A^-$ das Anion einer starken organischen oder anorganischen Säure ist,

$XN^+$ durch R', $R^2$ und $R^3$ substituiertes Pyridinium, Pyrimidinium, Thiazolium oder Imidazolium,

n, q und r die Zahl 1 oder 0 und p eine ganze Zahl zwischen 1 und 15,

Y die Gruppe $CH_2$, C(H,OH) oder C(O),

Z O, S, $CH_2$, C(O), NQ', $SO_2$, C(O)O, OC(O), C(O)N(Q') oder N(Q')C(O),

L durch $R^4$ substituiertes p-Phenylen und

M durch $R^5$ und $R^6$ substituiertes Phenyl ist,

T eine der für Z möglichen Bedeutungen hat oder $C(CH_3)_2$ $C_2H_4$, $C(Q^2)=C(Q^3)$, C≡C, $CH_2C(O)$, $C(O)CH_2$, $CH_2O$ oder $OCH_2$ ist,

R' eine Gruppe Ar, Ar-$C_{1-4}$ -Alkyl, ArO oder ArC(O),

Ar durch $R^7$, $R^8$ und $R^9$ substituiertes Phenyl,

$R^2$ und $R^3$ H, $C_{1-4}$-Alkyl, $C_{1-4}$-Alkoxy oder $C_6H_5$, wobei das N-Atom in 2-Stellung einer Imidazoliumgruppe $XN^+$ durch Ar, Ar-$C_{1-4}$-Alkyl oder $C_{1-4}$-Alkyl substituiert sein muss,

$R^4$ bis $R^9$ H, Halogen, $CF_3$, $NO_2$, CN, $C_{1-4}$-Alkyl, -Alkoxy, --Alkylthio oder -Alkylsulfonyl), $SO_2N(R,Q)$, $C(O)N(Q^4,Q^5)$, C-(O)$Q^4$, C(O)O$Q^4$ oder OC(O)$Q^4$,

R, Q, Q', $Q^2$ und $Q^3$ H oder $C_{1-4}$-Alkyl, und

$Q^4$ und $Q^5$ $C_{1-4}$-Alkyl sind,

hemmen die intestinale Resorption des Cholesterins und der Gallensalze im enterohepatischen Kreislauf. Man kann diese ein quaternäres N-Atom erhaltende Salze ausgehend von entsprechenden Aminen herstellen.

EP 0 189 094 A2

# Polycyclische Salze

Die vorliegende Erfindung betrifft neue polycyclische Salze, Verfahren zu ihrer Herstellung und Arzneimittel auf der Basis dieser Salze.

Die Salze haben die Formel

$A^- XN^+-CH_2-(Y)_n-(CH_2)_p-(Z)_q-L-(T)_r-M$ I

worin

$A^-$ das Anion einer starken organischen oder anorganischen Säure ist,

$XN^+$ durch $R^1$, $R^2$ und $R^3$ substituiertes Pyridinium, Pyrimidinium, Thiazolium oder Imidazolium,

n, q und r die Zahl 1 oder 0 und p eine ganze Zahl zwischen 1 und 15,

Y die Gruppe $CH_2$, C(H,OH) oder C(O),

Z O, S, $CH_2$, C(O), NQ', $SO_2$, C(O)O, OC(O), C(O)N(Q') oder N(Q')C(O),

L durch $R^4$ substituiertes p-Phenylen und

M durch $R^5$ und $R^6$ substituiertes Phenyl ist,

T eine der für Z möglichen Bedeutungen hat oder $C(CH_3)_2$, $C_2H_4$, $C(Q^2)=C(Q^3)$, C≡C, $CH_2C(O)$, $C(O)CH_2$, $CH_2O$ oder $OCH_2$ ist,

R' eine Gruppe Ar, Ar-$C_{1-4}$-Alkyl, ArO, oder ArC(O),

Ar durch $R^7$, $R^8$ und $R^9$ substituiertes Phenyl,

$R^2$ und $R^3$ H, $C_{1-4}$-Alkyl, $C_{1-4}$ -Alkoxy oder $C_6H_5$, wobei das N-Atom in 2-Stellung einer Imidazoliumgruppe $XN^+$ durch Ar, Ar-$C_{1-4}$-Alkyl oder $C_{1-4}$-Alkyl substituiert sein muss,

$R^4$ bis $R^9$ H, Halogen, $CF_3$, $NO_2$, CN, $C_{1-4}$-(Alkyl, -Alkoxy, --Alkylthio oder -Alkylsulfonyl), $SO_2N(R,Q)$, $C(O)N(Q^4,Q^5)$, $C(O)Q^4$, $C(O)OQ^4$ oder $OC(O)Q^4$,

R, Q, Q' $Q^2$ und $Q^3$ H oder $C_{1-4}$-Alkyl, und $Q^4$ und $Q^5$ $C_{1-4}$--Alkyl sind.

Die hier erwähnten mit $C_{1-4}$ bezeichneten Reste sind geradkettig oder verzweigt. Beispiele davon sind Methyl, Aethyl, Isopropyl, Methoxy, Methylthio und Methylsulfonyl. Beispiele für Anionen starker organischer oder anorganischer Säuren sind $C_{1-4}$-Alkyl- oder Phenylsulfonyloxy, Tosyloxy, bzw. $Cl^-$, $Br^-$, $J^-$, $ClO_4^=$, $SO_4^=$, $PO_4^{\equiv}$ und $NO_3^-$.

Die Salze der Formel I können hydratisiert sein. Die Hydratisierung kann im Zuge des Herstellungsverfahrens erfolgen oder allmählich als Folge hygroskopischer Eigenschaften eines zunächst wasserfreien Salzes der Formel I auftreten. Die Salze der Formel I können ferner ein oder mehrere asymmetrische C-Atome enthalten und somit als optisch aktive Enantiomere, als Diastereomere oder als Gemische davon, z.B. als Racemate, vorliegen.

Bevorzugte Salze der Formel I sind diejenigen, worin $XN^+$ in para-Stellung durch R' substituiertes Pyridinium ist, insbesondere diejenigen, worin $A^-$, $Cl^-$ oder $Br^-$; $XN^+$ p--Phenyl- oder p-Benzoylpyridinium; p die Zahl 1, 2, 8 oder 11; q die Zahl 1; n und r die Zahl 1 oder 0; Y CHOH; Z O, S, NH oder C(O)NH; $R^4$, $R^5$ und $R^6$ H; oder $R^4$ $CH_3$; $R^5$ F, Cl, Br, $CF_3$, $NO_2$, CN, $OCH_3$ oder $SO_2CH_3$ in para-Stellung zu $(T)_r$; und T C(O), O, S, $SO_2$, $CH_2$, $C_2H_4$, NH, $C(O)CH_2$ oder NHC(O) sind.

Beispiele solcher bevorzugten Salze sind folgende:

1-[2-Hydroxy-3-[p-(p-nitrobenzoyl)phenoxy]propyl]-4--phenylpyridiniumchlorid,

1-[12-(p-Phenäthylphenoxy)dodecyl]-4--phenylpyridiniumchlorid,

1-[3-[p-[(p-Chlorphenyl)thio]phenoxy]-2-hydroxypropyl]-4--phenylpyridiniumchlorid,

1-[3-[p-(p-Cyanobenzoyl)phenoxy]-2-hydroxypropyl]-4--phenylpyridiniumchlorid und

1-[9-(p-Phenäthylphenoxy)nonyl]-4-phenylpyridiniumbromid.

Die Salze der Formel I können dadurch hergestellt werden, dass man

a) das dem Kation $XN^+$ entsprechende substituierte aromatische Amin XN mit einer Verbindung der Formel

$G-CH_2-(Y)_n-(CH_2)_p-(Z)_q-L-(T)_r-M$ II

worin G eine Abgangsgruppe ist oder mit einer Hydroxymethylengruppe Y ein Epoxyd bildet,

umsetzt, oder

b) ein Pyryliumsalz der Formel

$A'^- X'O^+$ III

worin $A'^-$ ein Anion $BF_4^-$, $ClO_4^-$ oder $PF_6^-$, und $X'O^+$ durch R', $R^2$ und $R^3$ substituiertes Pyrylium ist,

mit einem Amin der Formel

$H_2N-CH_2-(Y)_n-(CH_2)_p-(Z)_q-L-(T)_r-M$ IV

worin $XN^+$, n, p, q, r, Y, Z, T, L und M die oben angegebene Bedeutung haben,

umsetzt und in dem erhaltenen Salz das Anion $A'^-$ durch $A^-$ ersetzt.

Beispiele von in den Verbindungen der Formel II enthaltenen Abgangsgruppen G sind Chlor, Brom, Jod, Mesyloxy, Phenylsulfonyloxy und Tosyloxy. Die Reaktion eines Amins XN mit einem eine Abgangsgruppe enthaltenden Verbindung II kann man bei einer Temperatur bis zur Rückflusstemperatur des Reaktionsgemisches durchführen, zweckmässig bei etwa 80°C oder bei Raumtemperatur, je

nach eingesetzten Reaktanten, gegebenenfalls in einem Lösungsmittel, wie einem aromatischen Kohlenwasserstoff, z.B. Toluol oder Benzol, oder Dimethylformamid (DMF) oder Acetonitril.

Die Reaktion eines Amins XN mit einem Epoxyd II kann man in einem Lösungsmittel, wie Wasser und Dioxan oder Tetrahydrofuran (THF), in saurem Medium (pH <8), zweckmässig in Gegenwart der dem Anion $A^-$ entsprechenden Säure, bei einer Temperatur bis zur Rückflusstemperatur des Reaktionsgemisches, zweckmässig bei etwa 70°C, durchführen.

Die Reaktion eines Pyryliumsalzes III mit einem Amin IV kann man bei einer Temperatur von 0°C bis zu Raumtemperatur durchführen, gegebenenfalls in einem Lösungsmittel, wie Toluol oder DMF.

Die Verbindungen der Formel II, worin $(Z)_q$ eine Gruppe Z' der Bedeutung O, S, N(Q') oder OC(O) ist, kann man durch Umsetzung einer Verbindung der Formel

$G\text{-}CH_2\text{-}(Y)_n\text{-}(CH_2)_p\text{-}G'$ V

worin G' eine Abgangsgruppe ist,

mit einer Verbindung der Formel

$HZ'\text{-}L\text{-}(T)_r\text{-}M$ VI

herstellen.

So kann man zur Herstellung eines Halogenids der Formel II, wie eines Bromids, worin z.B. $(Z)_q$ Sauerstoff ist, ein Dibromid der Formel V mit einem Phenol der Formel VI in Gegenwart einer Base, wie Natriumhydroxyd, in einem Lösungsmittel, wie Aethanol, unter Erhitzen, z.B. auf 80°C, umsetzen. Zur Herstellung eines entsprechenden Epoxyds der Formel II kann man ein Chlorepoxyd der Formel V, z.B. Epichlorhydrin, mit einem Phenol VI in Gegenwart einer Base, wie Piperidin, bei erhöhter Temperatur umsetzen. Zur Herstellung eines Epoxyds der Formel II, worin z.B. $(Z)_q$ die Gruppe NH ist, kann man ein Chlorepoxyd V mit einem Anilinderivat der Formel VI in Gegenwart von Kaliumcarbonat bei erhöhter Temperatur, z.B. unter Rückfluss, erhitzen.

Die Verbindungen der Formel II, worin Y Hydroxymethylen, Z Sauerstoff und G Mesyloxy, Phenylsulfonyloxy oder Tosyloxy sind, lassen sich auch ausgehend von den entsprechenden Diolen der Formel II herstellen, worin G für OH stehen würde. Diese Diolen kann man durch Umsetzung der entsprechenden Phenolen der Formel VI mit einem 3-Tosyloxy-1,2-propandiolacetonid und Aufspaltung des erhaltenen Dioxolans herstellen, z.B. wie beschrieben im Beispiel 7.

Die Verbindungen der Formel II, worin $(Z)_q$ S ist kann man z.B. mittels m-Chlorperbenzoesäure oder Wasserstoffperoxyd und Essigsäure zur entsprechenden eine Sulfonylgruppe $(Z)_q$ enthaltenden Verbindung II oxydieren.

Eine Verbindung der Formel II, worin $(Z)_q$ eine Gruppe N(Q')C(O) ist, kann man durch Umsetzung eines Amins der Formel

$G\text{-}CH_2\text{-}(Y)_n(CH_2)_p\text{-}N(H,Q')$ VII

mit einem Säurechlorid der Formel

$Cl\text{-}C(O)\text{-}L\text{-}(T)_r\text{-}M$ VIII

herstellen, z.B. wie beschrieben für die Umsetzung eines Amins XN mit einer eine Abgangsgruppe enthaltenden Verbindung II.

Zur Herstellung einer Verbindung der Formel II, worin $(Z)_q$ eine Gruppe C(O)O oder C(O)N(Q') ist, kann man in an sich bekannter Weise eine Säure der Formel

$G\text{-}CH_2\text{-}(Y)_n\text{-}(CH_2)_p\text{-}COOH$ IX

oder ein funktionelles Derivat davon, z.B. ein Säurechlorid oder ein Säureanhydrid, entweder mit einem Phenol der Formel

$HO\text{-}L\text{-}(T)_r\text{-}M$ X

verestern, oder mit einem Amin der Formel $(H,Q')N\text{-}L\text{-}(T)_r\text{-}M$ XI

umsetzen. So lässt sich z.B. das Chlorid einer Säure der Formel IX, z.B. das 3-Chlorpropionylchlorid, mit einem Amin der Formel XI in einem Lösungsmittel, wie Chloroform, in Gegenwart von einer Natriumhydroxydlösung zu einem Amid der Formel II umsetzen, worin $(Z)_q$ die Gruppe CONH ist.

Die Verbindungen der Formel II, worin $(Z)_q$ eine Carbonyl- oder Methylengruppe ist, kann man in an sich bekannter Weise herstellen, z.B. durch Umsetzung eines Aldehyds der Formel

$HC(O)\text{-}L\text{-}(T)_r\text{-}M$ XII

mit 1,3-Propandithiol und mit Butyllithium, Umsetzung des erhaltenen Lithiumsalzes mit einem Halogenid der Formel V, worin G' Halogen darstellt, und Ueberführung des erhaltenen Thioketals der Formel

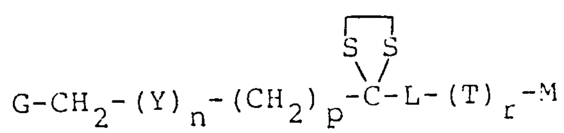

XIII

in das entsprechende Keton der Formel II, worin $(Z)_q$Carbonyl ist, mittels Quecksilber(II)-sulfat und Methanol, oder in die entsprechende Verbindung der Formel II, worin $(Z)_q$ Methylen ist, durch Hydrierung in Gegenwart von Raney-Nickel.

Die Pyryliumsalze der Formel III kann man in an sich bekannter Weise herstellen, z.B. ausgehend von den entsprechenden α- oder γ-Pyronen oder den entsprechenden 1,5-Diketonen oder 1,5-Dialdehyden.

Die Amine der Formel IV kann man in an sich bekannter Weise herstellen, z.B. durch Umsetzung eines Halogenids der Formel II mit Phthalimidkalium in Dimethylformamid und Umsetzung des erhaltenen Phthalimids mit Hydrazinhydrat in Aethanol.

Die Salze der Formel I besitzen wertvolle pharmakologische Eigenschaften. Sie hemmen insbesondere die intestinale Resorption des Cholesterins und der Gallensalze im entero-hepatischen Kreislauf. Dementsprechend kann man sie bei der Bekämpfung bzw. Verhütung von Krankheiten, wie Hypercholesterinämie, Atherosklerose und Fettsucht, verwenden.

Die Hemmung der intestinalen Resorption von Cholesterin lässt sich tierexperimentell wie folgt nachweisen:

Totenkopfäffchen werden die zu untersuchenden Substanzen zusammen mit einem Protein, Stärke, Triolein und -[26-$^{14}$C]-Cholesterin enthaltenden Futter oral verabreicht. Hierauf wird der Kot während 2,5 Tagen gesammelt. Die im Kot ermittelte Differenz zwischen dem verabreichten und dem ausgeschiedenen radioaktiven Cholesterin wird als Mass für resorbiertes Cholesterin genommen. Die Cholesterinresorption (CHORES) wird in Prozenten der vor der Medikation ermittelten Kontrollwerte ausgedrückt. In der nachstehenden Tabelle werden die Resultate wiedergegeben, welche mit einigen repräsentativen erfindungsgemässen Produkten erhalten worden sind. Angegeben werden für jede der darin figurierenden Verbindungen die verabreichte Dosis (in µMol/kg p.o.) sowie die jeweils ermittelte Cholesterinresorption (CHORES) in Prozenten der Cholesterinresorption in der Vorperiode. Ausserdem enthält die Tabelle Angaben über die akute Toxizität der untersuchten Verbindungen (Toxizität nach einmaliger oraler Verabreichung an Mäusen).

Tabelle

| Verbindung der Formel I in Beispiel Nr. | Dosis in µMol/kg p.o. | CHORES in % der Vorperiode | Toxizität in mg/kg p.o. |
|---|---|---|---|
| 1 | 30 | 31 | 5000 |
| 2A)e) | 30 | 45 | 5000 |
| 2A)o) | 30 | 44 | 5000 |
| 4A)d) | 30 | 47 | |
| 4A)e) | 30 | 39 | |

Die erfindungsgemässen Produkte können als Heilmittel, z.B. in Form pharmazeutischer Präparate, Verwendung finden. Die pharmazeutischen Präparate werden oral, z.B. in Form von Tabletten, Lacktabletten, Dragées, Hart- und Weichgelatinekapseln, Lösungen, Emulsionen oder Suspensionen, verabreicht.

Zur Herstellung von pharmazeutischen Präparaten können die erfindungsgemässen Produkte mit pharmazeutisch inerten, anorganischen oder organischen Trägern verarbeitet werden. Als solche Träger kann man für Tabletten, Lacktabletten, Dragées und Hartgelatinekapseln, z.B. Lactose, Maisstärke, Talk, Stearinsäure oder deren Salze verwenden. Für Weichgelatinekapseln eignen sich als Träger z.B. pflanzliche Oele, Wachse, Fette, halbfeste und flüssige Polyole; je nach Beschaffenheit des Wirkstoffes sind jedoch bei Weichgelatinekapseln überhaupt keine Träger erforderlich. Zur Herstellung von Lösungen und Sirupen eignen sich als Träger z.B. Wasser, Polyole, Saccharose, Invertzucker und Glukose.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt sind Arzneimittel, enthaltend ein Salz der Formel I ebenfalls Gegenstand der vorliegenden Erfindung, weiterhin auch ein Verfahren zur Herstellung solcher Arzneimittel, welches dadurch gekennzeichnet ist, dass man eine oder mehrere erfindungsgemässe Produkte und gegebenenfalls einen oder mehrere andere therapeutisch wertvolle Stoffe in eine galenische Darreichungsform bringt.

Die Dosierung kann innerhalb weiter Grenzen variieren und ist natürlich in jedem einzelnen Fall den individuellen Gegebenheiten anzupassen. Im Allgemeinen dürfte bei oraler Verabreichung eine Tagesdosis von etwa 50 mg bis etwa 3 g, vorzugsweise von etwa 200 mg bis 1 g angemessen sein.

Beispiel 1

Eine Lösung von 2,06 g (6,88 mMol)4-(2,3--Epoxypropoxy)-4'-nitrobenzophenon in 50 ml Dioxan und 25 ml Wasser wurde mit 1,06 g (6,88 mMol) 4--Phenylpyridin versetzt und bei 70°C unter Zugabe von 1N HCl bei pH <8 gerührt. Nach 18 Stunden Rühren bei 70°C wurde das Gemisch auf 20°C abgekühlt, mit 300 ml Wasser versetzt und dreimal mit 200 ml Chloroform extrahiert. Die organische Phase wurde getrocknet und eingeengt. Der Rückstand wurde aus Aceton umkristallisiert. Man erhielt 1--[2-Hydroxy-3-[p-(p-nitrobenzoyl)phenoxy]propyl]-4--phenylpyridiniumchlorid in 50% Ausbeute, Smp. 240°C.

Das Ausgangsepoxyd (Smp. 120-122°C) wurde analog Beispiel 2B)a) hergestellt.

Beispiel 2

2A) Analog Beispiel 1 wurden hergestellt:

2Aa) 1-[3-[p-(p-Fluorbenzoyl)phenoxy]-2-hydroxypropyl]-4-phenylpyridiniumchlorid, Smp. 234°C

2A)b) 1-[3-[p-(p-Chlorbenzoyl)phenoxy]-2-hydroxypropyl]-4-phenylpyridiniumchlorid, Smp. 220°C

2A)c) 1-[3-[p-(p-Brombenzoyl)phenoxy]-2-hydroxypropyl]-4-phenylpyridiniumchlorid, Smp. 180°C

2A)d) 1-[3-[p-(p-Anisoyl)phenoxy]-2-hydroxypropyl]-4-phenylpyridiniumchlorid, Smp. 207°C

2A)e) 1-[3-[p-(p-Cyanobenzoyl)phenoxy]-2-hydroxypropyl]-4-phenylpyridiniumchlorid, Smp. 245°C

2A)f) 1-[3-[(1-Benzoyl-o-tolyl)-oxy]-2-hydroxypropyl]-4-phenylpyridiniumchlorid, Smp. 192°C

2A)g) 1-[3-(p-Benzoylphenoxy)-2-hydroxypropyl]-4-phenylpyridiniumchlorid, Smp. 201°C

2A)h) 1-[3-[p-(Methylsulfonyl)benzoyl]phenoxy]-2-hydroxypropyl]-4-phenylpyridiniumchlorid, Smp. 148°C

2A)i) 1-[3-[p-(p-Chlorbenzoyl)anilino]-2-hydroxypropyl]-4-phenylpyridiniumchlorid, Smp. 248°C

2A)j) 1-[3-[[p-(p-Chlorbenzoylphenyl]thio]-2-hydroxypropyl]-4-phenylpyridiniumchlorid, Smp. 229°C

2A)k) 1-[3-(p-Benzylphenoxy)-2-hydroxypropyl]-4-phenylpyridiniumchlorid, Smp. 220°C

2A)l) 1-[3-[p-(p-Chlorphenoxy)phenoxy]-2-hydroxypropyl]-4-phenylpyridiniumchlorid, Smp. 208°C

2A)m) 1-[3-(p-Anilinophenoxy)-2-hydroxpropyl]-4-phenylpyridiniumchlorid, Smp. 216°C

2A)n) 1-[2-Hydroxy-3-[p-[(p-nitrophenyl)thio]phenoxy]-propyl]-4-phenylpyridiniumchlorid, Smp. 234°C

2A)o) 1-[3-[p-[(p-Chlorphenyl)thio]phenoxy]-2-hydroxypropyl]-4-phenylpyridiniumchlorid, Smp. 187°C

2A)p) 1-[3-[p-[(p-Bromphenyl)sulfonyl]phenoxy]-2-hydroxypropyl]-4-phenylpyridiniumchlorid, Smp. 223°C

2A)q) 1-[3-[p-(p-Chlorbenzamido)phenoxy]-2-hydroxypropyl]-4-phenylpyridiniumchlorid, Smp. 216°C

2A)r) 1-[2-Hydroxy-3-(p-phenacylphenoxy)propyl]-4-phenylpyridiniumchlorid, Smp. 249°C

2A)s) 1-[3-(4-Biphenylyloxy)-2-hydroxypropyl]-4-phenylpyridiniumchlorid, Smp. 204°C.

2B)a) Das zur Herstellung des obigen Produktes von Beispiel 2A)f) verwendete Ausgangsepoxyd wurde wie folgt hergestellt:

10,6 g 4-Hydroxy-3-methylbenzophenon, 50 g Epichlorhydrin und 3 Tropfen Piperidin wurden 12 Stunden auf dem Dampfbad erhitzt. Die Reaktionslösung wurde unter vermindertem Druck vom überschüssigen Epichlorhydrin befreit. Der Rückstand wurde in 20 ml Chloroform gelöst und nach Zugabe von 20 ml 3N Natronlauge bei Raumtemperatur geschüttelt. Die organische Phase wurde abgetrennt, mit Wasser gewaschen und zur Trockene eingedampft. Aus dem öligen Rückstand kristallisierte 4-(2,3-Epoxypropoxy)-3-methylbenzophenon, Smp. nach Umkristallisation aus Aethanol 56-58°C.

2B)b) Analog Beispiel 2B)a) wurden die folgenden Ausgangsepoxyde für die Beispiele 2A)e) bis 2A)r) hergestellt:

p-[p-(2,3-Epoxypropoxy)benzoyl]benzonitril, Smp. 100-101°C, Biespiel 2A)e)

4-(2,3-Epoxypropoxy)-4'-(methylsulfonyl)benzophenon, Smp. 165-167°C, Beispiel 2A)h)

4-Chlor-4'-[(2,3-epoxypropyl)thio]benzophenon, Smp. 94-95°C, Beispiel 2A)j)

1-[p-(Benzyloxy)phenoxy]-2,3-epoxypropan, Smp. 113-114°C (Methanol), Beispiel 2A)k)

1-[p-(p-Chlorphenoxy)phenoxy]-2,3-epoxypropan, Smp. 49-52°C (Aethylacetat-Petroläther), Beispiel 2A)1)

2,3-Epoxypropyl-p-)p-nitrophenylthio)phenyläther, Smp. 96-99°C (Methanol-Chloroform), Beispiel 2A)n)

1-[p-(p-Chlorphenylthio)phenoxy]-2,3-epoxypropan, Smp. 61-62°C (Isopropyläther), Beispiel 2A)o)

p-[(p-Bromphenyl)sulfonyl]phenyl-2,3-epoxypropyläther, Smp. 126-128°C (Isopropanol), Beispiel 2A)p)

p-Chlor-4'-(2,3-epoxypropoxy)benzanilid, Smp. 181°C - (Isopropanol), Beispiel 2A)q)

4'-(2,3-Epoxypropoxy)-2-phenylacetophenon, Smp. 85-86°C (Isopropanol), Beispiel 2A)r).

2B)c) Das zur Herstellung des Produktes von Beispiel 2A)i) verwendete Ausgangseproxyd wurde wie folgt hergestellt:

23,2 g 4-Chlor-4'-aminobenzophenon, 92 g Epichlorhydrin und 7 g Kaliumcarbonat wurden 15 Stunden am Rückfluss erhitzt. Das überschüssige Epichlorhydrin wurde im Vakuum verdampft, der feste Rückstand wurde zwischen Wasser und Chloroform verteilt. Aus der organischen Phase erhielt man 4-Chlor-4'-[(2,3-epoxypropyl)amino]-benzophenon, das zweimal aus Methanol umkristallisiert wurde, Smp. 128-129°C.

Beispiel 3

Eine Lösung von 3 g (9,39mMol)1-(3-Brompropoxy)-4--(2-phenäthyl)benzol und 5 g 4-Phenylpyridin in 60 ml Toluol wurde 24 Stunden bei 100°C erhitzt und dann auf 20°C gekühlt. Nach Filtrierung und Umkristallisation aus Methylenchlorid-Aceton erhielt man 1-(3-(p--Phenäthylphenoxy)propyl]-4-phenylpyridiniumbromid in 70% Ausbeute, Smp. 128°C.

Das Ausgangsbromid wurde wie folgt hergestellt:

Eine Lösung von 1,4 g p-(2-Phenäthyl)phenol in 10 ml Aethanol wurde zu einer Lösung von 0,282 g Natriumhydroxid in 10 ml Aethanol gegeben. Nach Zugabe von 1,44 ml 1,3-Dibrompropan wurde 2 Stunden bei 80°C erhitzt, auf Raumtemperatur abkühlen gelassen und filtriert. Das Filtrat wurde am Rollverdampfer getrocknet. Der Rückstand wurde über Kieselgel mit Methylenchlorid gereinigt. Man erhielt einen farblosen öligen Rückstand, 1-(3--Brompropoxy)-4-(2-phenäthyl)benzol.

Beispiel 4

4A) Analog Beispiel 3 wurden hergestellt:

4A)a) aus 3-Phenylpyridin das

1-[3-(p-Phenäthylphenoxy)propyl]-phenylpyridiniumbromid, Smp.155°C

4A)b) aus 4-Phenylpyrimidin das

1-[3-(p-Phenäthylphenoxy)propyl] -4--phenylpyrimidiniumbromid, Smp. 169°C

4A)c) aus 4-Benzoylpyridin das

4-Benzoyl-1-[3-p-phenäthylphenoxy)propyl] pyridiniumbromid, Smp. 161°C

4A)d) aus 9-Bromnonyl-p-phenäthyl-phenyläther (Smp. 31°C) das

1-[9-(p-Phenyläthylphenoxy)nonyl] -4--phenylpyridiniumbromid, Smp. 147°C

4A)e) aus 12-Bromdodecyl-p-phenäthyl-phenyläther -(Smp.50-51°C) das

1-[12-(p-Phenäthylphenoxy)dodecyl] -4--phenylpyridiniumbromid, Smp. 120°C

4A)f) aus 1-[p-(3-Brompropoxy)phenyl]-2-phenyl-1-äthanon (Smp. 120°C) das

4-Phenyl-1-[3-[p-(phenacetyl)phenoxy]propyl] pyridiniumbromid, Smp. 189°C

4A)g) aus 4-(3-Brompropoxy)benzophenon (Smp. 75-76°C in Methanol) das

1-[3-(p-Benzoylphenoxy)propyl]-4-phenylpyridiniumbromid in Form eines Oels,

4A)h) aus 4-(3-Brompropoxy)-4-chlorbenzophenon -(Smp.73-75°C in Methanol) das

1-[3-[p-(p-Chlorbenzoyl)phenoxy]propyl] -4--phenylpyridiniumbromid, Smp. 211°C

4A)i) aus 4-(3-Chlorpropionamido)benzophenon das

1-[2-[(p-Benzoylphenyl)carbamoyl]äthyl] -4--phenylpyridiniumchlorid, Smp. 251°C

4A)j) aus 1-(3-Brompropoxy)-4-(p-chlorphenoxy)benzol -(Sdp. 155-160°C unter 0,08 Torr) das

1-[3-[p-(p-Chlorphenoxy)phenoxy]propyl] -4--phenylpyridiniumbromid, Smp. 172°C

4A)k) aus 4-(3-Brompropoxy)-4'-nitrobenzophenon das

1-[3-[p(p-Nitrobenzoyl)phenoxy]propyl] -4--phenylpyridiniumbromid, Smp. 222°C

4A)l) aus 4-(12-Bromdodecyloxy)-4'-nitrobenzophen das

1-[12-](p-Nitrobenzoyl)phenoxy]dodecyl] -4--phenylpyridiniumbromid, Smp. 126-127°C

4A)m) aus 4-(3-Brompropoxy)-4'-triflourmethylbenzophenon das

1-[3-[p-(p-Triflourmethylbenzoyl)phenoxy]propyl]-4--phenylpyridiniumbromid, Smp. 173-177°C.

4B) Das zur Herstellung des obigen Produktes von Beispiel 4A)i) verwendete Ausgangschlorid wurde wie folgt hergestellt:

Eine Lösung von 3,95 g p-Aminobenzophenon in 100 ml Chloroform wurde mit 2,6 g 3-Chlorpropionylchlorid versetzt und nach Zugabe von 26 ml 1N Natronlauge bei Raumtemperatur geschüttelt. Die organische Phase wurde abgetrennt, nach einander mit Wasser, 1N HCl und Wasser gewaschen, getrocknet und eingedampft. Nach Umkristallisation aus Methanol des erhaltenen Oels erhält man das 4--(3-Chlorpropionamido)benzophenon, Smp. 135-136°C.

Beispiel 5

Durch filtrierung des Bromidproduktes des Beispiels 3 durch einen Stark basischen mit Chlorionen geladenen Anionenaustauscher Styrol-Divinylbenzol (IRA 400) erhielt man das 1-[3-(p-Phenäthylphenoxy)propyl]-4--phenylpyridiniumchlorid in 95% Ausbeute, Smp. 126°C.

Beispiel 6

Analog Beispiel 5 erhielt man

6a) aus dem Bromid von Beispiel 4A)h) das 1-[3-[p-(p--Chlorbenzoyl)phenoxy]propyl] -4-phenylpyridiniumchlorid, Smp. 196°C

6b) aus dem Bromid von Beispiel 4A)k) das 1-[3-[-(p--Nitrobenzoyl)phenoxy]propyl] -4-phenylpyridiniumchlorid, Smp. 201-203°C

6c) aus dem Bromid von Beispiel 4A)1) das 1-[12-(p--Nitrobenzoyl)phenoxy]dodecyl] -4-phenylpyridiniumchlorid, Smp. 146°C

6d) aus dem Bromid von Beispiel 4A)m) das 1-[3-[p-(p--Trifluormethylbenzoyl) phenoxy] -4-phenylpyridiniumchlorid, Smp. 170-172°C.

Beispiel 7

Unter analogen Bedingungen wie im Beispiel 3 wurden 9,5 g (20, 15 mMol) (S)-2-Hydroxy-3-[p-(p-nitrobenzoyl)-phenoxy[propyl-p-toluolsulfonat mit 10 g 4-Phenylpyridin in 120 ml Toluol zu 4,6 g 1-[(R)-2-Hydroxy-3-[p-(p--nitrobenzoyl)phenoxy]propyl]-4-phenylpyridiniumchlorid, Smp. 238°C, $[\alpha]^{20}_{D}$ +43,8° (c 0,8%, MeOH) umgesetzt.

Das Ausgangstotsylat wurde wie folgt heregestellt:

a) 1 g 4-Hydroxy-4'-nitrobenzophenon wurde zu 110 mg NaH enthaltenden 8 ml DMF zugesetzt. Das Gemisch wurde 30 Minuten bei 40-50°C erhitzt, dann mit 1,29 g (R)-3-Tosyloxy-1,2-propandiolacetonid und einer Spur NaI versetzt und unter Argon 4 Stunden bei 150°C (Oelbad) erhitzt. Nach Aufarbeitung und Reinigung erhielt man 930 mg (64%) 4-[[(S)-2,2-Dimethyl-1,3-dioxolan -4-yl]methoxy]--4'-nitrobenzophenon, Smp. 92-93°C,$[\alpha]^{20}_{D}$ + 5,3°C (C 1%, MeOH).

b) 0.5 g des Dioxolans von a) in 5 ml THF wurden 0.5 ml einer 25%igen Salzsäurelösung zugesetzt. Nach 3 Stunden Rühren bei Raumtemperatur und Aufarbeiten erhielt man 420 mg (95%) 4-[(R)-2,3-Dihydroxypropoxy]-4'--nitrobenzophenon, Smp. 104-105°C, $[\alpha]^{20}_{D}$ - 7,17° (c 0,6%, MeOH).

c) 500 mg des Diols von b) in 7.5 ml Methylendichlorid wurden 0,75 ml Pyridin und 299 mg Tosylchlorid zugesetzt. Nach 24 Stunden Rühren bei Raumtemperatur und Aufarbeiten erhielt man 500 mg (67%) des erwünschten Tosylats, $[\alpha]^{20}_{D}$ + 4,99° (c 0,8%, MeOH).

Beispiel A

Tabletten mit folgender Zusammensetzung kann man in an sich bekannter Weise herstellen:

| | Inhalt pro Tablette |
|---|---|
| Salz der Formel I | 200 mg |
| Mikrokristalline Cellulose | 155 mg |
| Maisstärke | 25 mg |
| Talk | 25 mg |
| Hydroxypropylmethylcellulose | 20 mg |
| | 425 mg |

**Ansprüche**

1. Polycyclische Salze der Formel

$A^- XN^+-CH_2-(Y)_n-(CH_2)_p-(Z)_q-L-(T)_r-M$ I

worin

$A^-$ das Anion einer starken organischen oder anorganischen Säure ist,

$XN^+$ durch $R^1$,$R^2$ und $R^3$ substituiertes Pyridinium, Pyrimidinium, Thiazolium oder Imidazolium,

n, q und r die Zahl 1 oder 0 und p eine ganze Zahl zwischen 1 und 15,

Y die Gruppe $CH_2$, C(H,OH) oder C(O),

Z O, S, $CH_2$, C(O), NQ', $SO_2$, C(O)O, OC(O), C(O)N(Q') oder N(Q')C(O),

L durch $R^4$ substituiertes p-Phenylen und

M durch $R^5$ und $R^6$ substituiertes Phenyl ist,

T eine der für Z möglichen Bedeutungen hat oder $C(CH_3)_2$, $C_2H_4$, $C(Q^2)=C(Q^3)$, C≡C, $CH_2C(O)$, $C(O)CH_2$, $CH_2O$ oder $OCH_2$ ist,

$R^1$ eine Gruppe Ar, Ar-$C_{1-4}$-Alkyl, ArO oder ArC(O),

Ar durch $R^7$, $R^8$ und $R^9$ substituiertes Phenyl,

$R^2$ und $R^3$ H, $C_{1-4}$-Alkyl, $C_{1-4}$-Alkoxy oder $C_6H_5$, wobei das N-Atom in 2-Stellung einer Imidazoliumgruppe $XN^+$ durch Ar, AR-$C_{1-4}$ -Alkyl oder $C_{1-4}$-Alkyl substituiert sein muss,

$R^4$ bis $R^9$ H, Halogen, $CF_3$, $NO_2$, CN, $C_{1-4}$-(Alkyl, -Alkoxy, --Alkylthio oder -Alkylsulfonyl), $SO_2N(R,Q)$, $C(O)N(Q^4,Q^5)$, $C(O)Q^4$, $C(O)OQ^4$ oder $OC(O)Q^4$,

R, Q, Q', $Q^2$ und $Q^3$ H oder $C_{1-4}$-Alkyl, und $Q^4$ und $Q^5$ $C_{1-4}$-Alkyl sind.

2. Salze nach Anspruch 1, worin Y Hydroxymethylen ist.

3. Salze nach Anspruch 1 oder 2, worin $XN^+$ in para--Stellung durch $R^1$ substituiertes Pyridinium ist.

4. Salze nach Anspruch 1, 2 oder 3, worin $A^-$, $Cl^-$ oder $Br^-$; $XN^+$ p-Phenyl- oder p-Benzoylpyridinium: p die Zahl 1, 2, 8 oder 11; q die zahl 1; n und r Zahl 1 oder 0; Y CHOH; Z O, S, NH oder C(O)NH; $R^4$, $R^5$ und $R^6$ H; oder $R^4$ $CH_3$; $R^5$ F, Cl, Br, $CF_3$, $NO_2$, CN, $OCH_3$ oder $SO_2$ $CH_3$ in para--Stellung zu $(T)_r$ ; und T C(O), O, S, $SO_2$, $CH_2$, $C_2H_4$, NH,

$C(O)CH_2$ oder NHC(O) sind.

5. 1-[2-Hydroxy-3-[p-(p-nitrobenzoyl)phenoxy[propyl]-4--phenylpyridiniumchlorid.

6. Ein Salz aus der Gruppe der folgenden

1-[12-(p-Phenäthylphenoxy)dodecyl]-4--phenylpyridiniumchlorid,

1-[3-[p-(p-Chlorphenyl)thio]phenoxy]-2-hydroxypropyl]-4--phenylpyridiniumchlorid,

1-[3-[p-(p-Cyanobenzoyl)phenoxy]-2-hydroxypropyl]-4--phenylpyridiniumchlorid und

1-[9-(p-Phenäthylphenoxy)nonyl]-4-phenylpyridiniumbromid.

7. Salze nach einem der Ansprüche 1-6 als pharmazeutische, insbesondere die intestinale Resorption des Cholesterins und der Gallensalze im enterohepatischen Kreislauf hemmende Wirkstoffe.

8. Verfahren zur Herstellung der Salze der Formel I, nach einem der Ansprüche 1 6, dadurch gekennzeichnet, dass man

a) das dem Kation $XN^+$ entsprechende aromatische Amin XN mit einer Verbindung der Formel

$G-CH_2-(Y)_n-(CH_2)_p-(Z)_q-L-(T)_r-M$ II

worin G eine Abgangsgruppe ist oder mit einer Hydroxymethylengruppe Y ein Epoxyd bildet,

umsetzt, oder

b) ein Pyryliumsalz der Formel

$A'^- X'O^+$ III

Worin $A'^-$ ein Anion $BF_4^-$, $ClO_4^-$ oder $PF_6^-$, und $X'O^+$ durch R', $R^2$ und $R^3$ substituiertes Pyrylium ist,

mit einem Amin der Formel

$H_2N-CH_2-(Y)_n-(CH_2)_p-(Z)_q-L-(T)_r-M$ IV

worin $XN^+$, n, p, q, r, Y, Z, T, L und M die oben angegebene Bedeutung haben,

umsetzt und in dem erhaltenen Salz das Anion $A'^-$ durch $A^-$ ersetzt.

9. Pharmazeutisches Präparat auf der Basis eines Salzes der Formel I in einem der Ansprüche 1-6.

10. Verwendung eines Salzes nach einem der Ansprüche 1-6 bei der Herstellung eines pharmazeutischen Präparats nach Anspruch 9.

Patentansprüche für den Vertragsstaat: AT

1. Verfahren zur Herstellung von polycyclischen Salzen der Formel

$A^- XN^+-CH_2-(Y)_n-(CH_2)_p-(Z)_q-L-(T)_r-M$ I

worin

$A^-$ das Anion einer starken organischen oder anorganischen Säure ist,

$XN^+$ durch R', $R^2$ und $R^3$ substituiertes Pyridinium, Pyrimidinium, Thiazolium oder Imidazolium,

n, q und r die Zahl 1 oder 0 und p eine ganze Zahl zwischen 1 und 15,

Y die Gruppe $CH_2$, C(H,OH) oder C(O),

Z O, S, $CH_2$, C(O), NQ', $SO_2$, C(O)O, OC(O), C(O)N(Q') oder N(Q')C(O),

L durch $R^4$ substituiertes p-Phenylen und

M durch $R^5$ und $R^6$ substituiertes Phenyl ist,

T eine der für Z möglichen Bedeutungen hat oder $C(CH_3)_2$, $C_2H_4$, $C(Q^2)=C(Q^3)$,C≡C, $CH_2C(O)$, $C(O)CH_2$, $CH_2O$ oder $OCH_2$ ist,

R' eine Gruppe Ar, Ar-$C_{1-4}$-Alkyl, ArO oder ArC(O),

Ar durch $R^7$, $R^8$ und $R^9$ substituiertes Phenyl,

$R^2$ und $R^3$ H, $C_{1-4}$-Alkyl,$C_{1-4}$-Alkoxy oder $C_6H_5$, wobei das N-Atom in 2-Stellung einer Imidazoliumgruppe $XN^+$ durch Ar, Ar-$C_{1-4}$-Alkyl oder $C_{1-4}$-Alkyl substituiert sein muss,

$R^4$ bis $R^9$ H, Halogen, $CF_3$, $NO_2$, CN, $C_{1-4}$-(Alkyl, -Alkoxy, --Alkylthio oder -Alkylsulfonyl), $SO_2N(R,Q)$, $C(O)N(Q^4,Q^5)$, $C(O)Q^4$, $C(O)OQ^4$ oder $OC(O)Q^4$,

R, Q, Q', $Q^2$ und $Q^3$ H oder $C_{1-4}$-Alkyl, und

$Q^4$ und $Q^5$ $C_{1-4}$-Alkyl sind, dadurch gekennzeichnet, dass man

a) das dem Kation $XN^+$ entsprechende aromatische Amin XN mit einer Verbindung der Formel

$G-CH_2-(Y)_n-(CH_2)_p-(Z)_q-L-(T)_r-M$ II

worin G eine Abgangsgruppe ist oder mit einer Hydroxymethylengruppe Y ein Epoxyd bildet,

umsetzt, oder

b) ein Pyryliumsalz der Formel

$A'^- X'O^+$ III

worin $A'^-$ ein Anion $BF_4^-$, $ClO_4^-$ oder $PF_6^-$, und $X'O^+$ durch R', $R^2$ und $R^3$ substituiertes Pyrylium ist,

mit einem Amin der Formel

$H_2N\text{-}CH_2\text{-}(Y)_n\text{-}(CH_2)_p\text{-}(Z)_q\text{-}L\text{-}(T)_r\text{-}M$ IV

worin $XN^+$, n, p, q, r, Y, Z, T, L und M die oben angegebene Bedeutung haben,

umsetzt und in dem erhaltenen Salz das Anion $A'^-$ durch $A^-$ ersetzt.

2. Verfahren nach Anspruch 1, worin Y Hydroxymethyl ist.

3. Verfahren nach Anspruch 1 oder 2, worin $XN^+$ in p-Stellung durch R' substituiertes Pyridinium ist.

4. Verfahren nach Anspruch 1, 2 oder 3, worin $A^-$, $Cl^-$ oder $Br^-$; $XN^+$ p-Phenyl- oder p-Benzoylpyridinium; p die Zahl 1, 2, 8 oder 11; q die zahl 1; n und r die Zahl 1 oder 0; Y CHOH; Z O, S, NH oder C(O)NH; $R^4$, $R^5$ und $R^6$ H; oder $R^4$ $CH_3$; $R^5$ F, Cl, Br, $CF_3$, $NO_2$, CN, $OCH_3$ oder $SO_2CH_3$ in para-Stellung zu $(T)_r$; und T C(O),O, S, $SO_2$, $CH_2$, $C_2H_4$, NH, $C(O)CH_2$ oder NHC(O) sind.

5. Verfahren nach einem der Ansprüche 1-4 zur Herstellung von 1-[2-Hydroxy-3[p-(p-nitrobenzoyl)phenoxy]propyl]-4-phenylpyridiniumchlorid, dadurch gekennzeichnet, dass man entsprechend substituierte Ausgangsverbindungen einsetzt.

6. Verfahren nach Anspruch 1 zur Herstellung eines Salzes aus der Gruppe der folgenden:

1-[12-(p-Phenäthylphenoxy)dodecyl]-4-phenylpyridiniumchlorid,

1-[3-[p-[(p-Chlorphenyl)thio]phenoxy]-2-hydroxypropyl]-4-phenylpyridiniumchlorid,

1-[3-[p-(p-Cyanobenzoyl)phenoxy]-2-hydroxypropyl]-4-phenylpyridiniumchlorid und

1-[9-(p-Phenäthylphenoxy)nonyl]-4-phenylpyridiniumbromid, dadurch gekennzeichnet, dass man entsprechend substituierte Ausgangsverbindungen einsetzt.